(19) Europäisches Patentamt
European Patent Office
Office européen des brevets

(11) Publication number: 0 100 217
B1

(12) EUROPEAN PATENT SPECIFICATION

(45) Date of publication of patent specification: 07.10.87

(51) Int. Cl.⁴: C 12 Q 1/26

(21) Application number: 83304262.5

(22) Date of filing: 22.07.83

# (54) Process for quantitative determination of substrate treated with oxidase.

(30) Priority: 23.07.82 JP 128700/82
29.09.82 JP 170639/82
20.06.83 JP 110423/83

(43) Date of publication of application:
08.02.84 Bulletin 84/06

(45) Publication of the grant of the patent:
07.10.87 Bulletin 87/41

(84) Designated Contracting States:
AT BE CH DE FR GB IT LI LU NL SE

(56) References cited:
EP-A-0 004 857
DE-B-2 439 348
DE-B-2 648 759
DE-B-2 913 553

(73) Proprietor: WAKO PURE CHEMICAL INDUSTRIES, LTD.
10, Doshomachi-3-chome
Higashi-ku Osaka (JP)

(72) Inventor: Sakata, Yoshitsugu
36-7, Hiyoshidai-2-chome
Otsu-shi (JP)
Inventor: Miyashita, Yoshinobu
14-17, Takadono-7-chome
Asahi-ku Osaka (JP)
Inventor: Hamanaka, Tadashi
52-6-506, Tsuchiyamacho
Nada-ku Kobe (JP)
Inventor: Kodera, Hiroyuki
83-2, Higashisonodacho-6-chome
Amagasaki-shi (JP)
Inventor: Miki, Yutaka
Mogawaryo, 3-10, Takadacho
Amagasaki-shi (JP)
Inventor: Yamanishi, Kazuhiko
17-10, Akatsuka-3-chome
Itabashi-ku Kobe (JP)
Inventor: Hanada, Toshiro
Minamihaitsu 103-go 784, Oaza Minamiotsuka
Kawagoe-shi (JP)

EP 0 100 217 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(74) Representative: **Baillie, Iain Cameron et al**
**c/o Ladas & Parry Isartorplatz 5**
**D-8000 München 2 (DE)**

**Description**

This invention relates to a process for quantitatively determining a substrate. More particularly, this invention relates to a process for quantitatively determining a substrate such as blood components applying an enzymic reaction in clinical chemical examinations and the like.

In quantitative determination of substrates by means of enzymes, particularly oxidases, the products by the enzymic reactions are water, carbon dioxide gas and hydrogen peroxide. Recently, the measurement of the produced hydrogen peroxide for quantitative determination of substrates has gained wide applications due to biochemical knowledge that enzymes have inherent specificity, that is, quantitativeness. As a result, chemical quantitative determinations previously used are hardly employed, since various devices are necessary for maintaining quantitativeness, corrosion of chemicals causes fatal problems, there are some problems in specificity, and the like. But recent enzymic processes are not always sufficiently satisfactory. This can be explained referring to quantitative determination of cholesterol as follows.

An increase of cholesterol causes hypercholesterolemia which is found in nephrosis syndrome, serious diabetes mellitus, dysthryoidism, glycogen accumulation disease, familial hyperlipemia, and the like. On the other hand, a decrease of cholesterol causes hypocholesterinemia which is found in serious liver disease, insufficient nutrition, hyperthyroidism, and the like. The quantitative determination of cholesterol is an essential test item in the field of clinical chemical examinations. As processes for quantitatively determining cholesterol, there were employed Zak-Henry's method and Zurkowsky method wherein the Liebermann-Burchard reaction and the Kiliani reaction were applied to a colorimetric reaction. But, after the proposal of a combination of an enzyme which oxidizes cholesterol to $\Delta^4$-cholestenon and hydrogen peroxide with a reagent for measuring the produced hydrogen peroxide, this enzymic process becomes a major process for quantative determination of cholesterol. But this enzymic process still requires further improvements, since influences of reducing substances in body fluid cannot be prevented. Further, the sensitivity is insufficient and the use of oxidizable color producing reagents which can produce color at higher wavelength sides is required for improvement.

DE 2,439,348B, DE 2,913,553B, EP 0,004,857A and EP 0,080,304A disclose processes and reagents for the detection or quantitative determination of substrates such as glucose, cholesterol and uric acid which oxidise the substrate. These methods and reagents rely on oxidation or oxidation-coupling of colour-producing reagents to enable the substrate to be detected or determined.

The present inventors had doubts about previous findings that enzymic reactions caused by individual specific oxidases for various substrates simply produce final products such as water and hydrogen peroxide and studied enzymic reactions extensively with a hope that new applications can be obtained by studying enzymic reactions. After studies of various combinations of substrates in body fluid and oxidases showing specificity, it was found that superoxide ions were produced quantatively by enzymic reactions of individual specific oxidases with substrates and said superoxide ions were changed to certain substances such as hydrogen peroxide and that the measurement of superoxide ions made it possible to determine quantitatively the substrates.

The present invention utilises the following reaction:

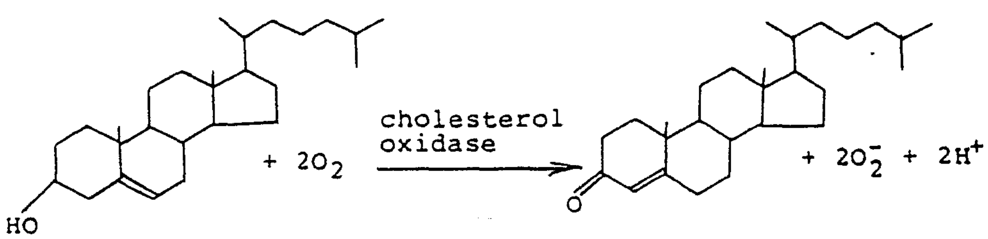


cholesterol $\Delta^4$-cholestenon

As shown above, superoxide ion ($O_2^-$) is produced by the enzymic action of cholesterol oxidase.

The produced superoxide ion is usually easily converted into hydrogen peroxide and this reaction is accelerated by superoxide dismutase usually present in blood serum (T. Matsuura: Synthetic Chemical Series, Enzymic Oxidation Reaction, page 69, Maruzen Co., Ltd. 1977). It is known that superoxide ion reacts with a reducible colour producing reagent to form a colour (Y. Oyanagi: Superoxides and Medical Science, pages 7—8, Kyoritsu Publishing Co. 1981), but the reaction is too slow to apply to quantitative determination of cholesterol in practical applications. It is stated in Roempp Lexicon (1981) p. 1511 that —SH transfer of glutathione plays an important role in biological redox systems.

In one aspect, the invention provides a process for determining a substrate, said process comprising treating a sample containing said substrate with a specific oxidase corresponding to said substrate to generate superoxide ion, and reducing a colour-producing reagent with said superoxide ion, characterised in that said process is a quantitative process which involves a colorimetric measurement of the reduced

colour-producing reagent and further involves treatment with a peroxidase, or an iron complex of porphyrin or an iron chelate of complexane, or phenol compound and/or amine, and a thiol compound.

In another aspect, the invention provides a composition for quantitative determination of a substrate comprising (a) a specific oxidase corresponding to a substrate to be determined, (b') iron complex or porphyrin or iron chelate of complexane, (c) an amine and/or a phenol, (d) a thiol compound and (e) a colour producing reagent reducible by superoxide ion.

In another aspect, the invention provides a composition for quantitative determination of a substrate comprising (a) a specific oxidase corresponding to a substrate to be determined, (b) a peroxidase, (c) a phenol and/or an amine, (d) a thiol compound, and (e) a colour producing reagent reducible by superoxide ion.

The thiol compound blocks the change of superoxide ion to hydrogen proxide and accelerates the reaction with the reducible colour producing reagent to be reduced to form a colour. However, the amine or phenol compound ensures that the determination is quantitative.

The invention is applicable to the quantitative determination of glucose, cholesterol, glycerol, glycerol phosphate, choline, acyl CoA, pyruvic acid, uric acid, xanthine and lactic acid for example.

The invention has certain advantages, namely that because a reducible producing reagent is used, there is no influence of reducing substances usually present in body fluid such as bilirubin and ascorbic acid. Also reducible colour-producing reagents are available which have high sensitivity and can produce colour in longer wavelength regions, and the like. Thus, the process of this invention is very advantageous in clinical examinations.

Thus, by measuring color formation or color change produced by reduction of a color producing reagent to be reduced by the superoxide ion, cholesterol in body fluid or in a sample can be determined quantitatively.

In the case of quantitative determination of cholesterol in body fluid sample, to a suitable medium containing body fluid to be tested, (a) cholesterol oxidase, (b) peroxidase, (c) a phenol, (d) a thiol compound, (e) a color producing reagent to be reduced such as Nitrotetrazolium Blue (hereinafter referred to as "$NO_2$—TB") are added and incubated, and a color produced is determined colorimetrically. That is, the cholesterol in the body fluid sample is treated with cholesterol oxidase and the generated superoxide ion is color formed by the color producing reagent to be reduced, and thus the cholesterol in the sample can be determined quantitatively.

During the above-mentioned examination, pH is maintained at 7.0 or higher, preferably 7.5 or higher.

As the color producing reagent to be reduced, tetrazolium compounds had various problems in that formazans produced by reduction of tetrazolium compounds are difficulty soluble in water, quantitativeness of color formation is not good, and devices are contaminated, and the like. But recent development obtained by introducing a group which improves the solubility into tetrazolium compounds solved such problems and makes it possible to employ these compounds in this invention.

Embodiments of this invention are explained in detail referring to the following experiments in conjunction with Figures 1 to 5 of the accompanying drawings.

In the accompanying drawings, Figs. 1 to 3 are graphs showing change of absorbance with the lapse of time, Fig. 4 shows calibration curves and Fig. 5 is a graph showing an absorption curve.

In order to confirm the production of superoxide ion by the enzymic action of cholesterol oxidase, the following experiments are conducted using cholesterol oxidase and $NO_2$—TB.

The reaction for producing superoxide ion by cholesterol oxidase and the confirmation reaction thereof are as follows:

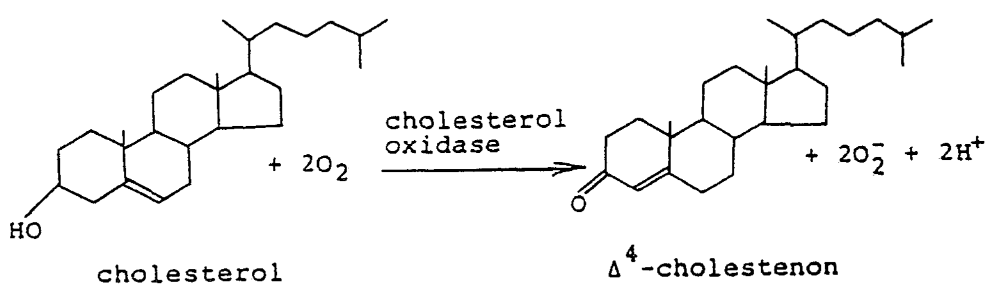


cholesterol $\Delta^4$-cholestenon

$$NO_2\text{—TB} + Cl^- + 2O_2^- + 2H^+ \rightarrow \tfrac{1}{2}\ \text{diformazan} + 2O_2 + HCl$$

(Reduction of $NO_2$—TB by superoxide ion + Formation of diformazan)

Experiment 1 (Qualitative test)

Using cholesterol oxidase and $NO_2$—TB, coloring by superoxide ion are tested qualitatively.

Further, influences of change of pH and of addition of 1-methoxy-phenazine metosulfate (1-methoxy PMS), diaphorase, or reduced glutathione (hereinafter referred to as "GSH") are tested.

*Reagents*

(1) Cholesterol solution

200 Mg of cholesterol is dissolved in isopropanol to make the volume 100 ml.

(2) Color producing reagent solution A

In 0.1 M tris (precisely tris-HCl) buffer solution (pH 8.0), 150 units of cholesterol oxidase and 50 mg of $NO_2$—TB are dissolved and the whole volume is made 1000 ml.

(3) Color producing reagent solution B

To the color producing reagent solution A, 100 mg of reduced glutathione is added and the whole volume is made 1000 ml.

(4) Color producing reagent solution C

To the color producing reagent solution A, 30 mg of 1-methoxy PMS is added and the whole volume is made 1000 ml.

(5) Color producing reagent solution D

To the color producing reagent solution A, 2000 units of diaphorase is added and the whole volume is made 1000 ml.

(6) Color producing reagent solution E

0.1 M tris buffer solution (pH 7.0) is used in the color producing reagent solution B in place of 0.1 M tris buffer solution (pH 8.0).

(7) Hydrogen peroxide solution

There is prepared a 0.1% hydrogen peroxide solution.

(8) Isopropanol (IPA)

*Experimental Method*

Each 50 µl of the cholesterol solution is placed in 5 test tubes and each 3 ml of the color producing reagent solutions A, B, C, D, and E is added to each test tube and incubated in a constant temperature chamber at 37°C for 10 minutes. Then, color formation is observed in comparison with the reagent blank. The results are as shown in Table 1.

TABLE 1

| Additive in color producing reagent solution | pH | Degree of coloring |
|---|---|---|
| None | 8 | x |
| GSH | 8 | o |
| 1-Methoxy PMS | 8 | x |
| Diaphorase | 8 | x |
| GSH | 7 | Δ |

Note) o: Color is produced.
x: Color is not produced.
Δ: Color is produced by weak.

As is clear from Table 1, the color is formed clearly only when GSH (reduced glutathione) is added and the reaction is conducted at pH 8.0.

Fig. 1 is a graph showing changes of absorbances with the lapse of time using the color producing reagent solution B and changing the amount of the cholesterol solution from 50 µl to 100 µl and 150 µl, subjected to the same reaction as mentioned above, and obtained by measuring the absorbances at wavelength of 560 nm.

Fig. 2 is a graph showing changes of absorbances with the lapse of time using the color producing reagent solution B and the hydrogen peroxide solution or IPA in place of the cholesterol solution, obtained in the same manner as in Fig. 1.

As is clear from Figs. 1 and 2, it is confirmed that the coloring is caused by the superoxide ion.

Experiment 2

Effects of Addition of Peroxidase, Phenol

*Reagents*

(1) Cholesterol solution

The same as in Experiment 1.

(2) Color producing reagent solution F

In 0.1 M tris buffer solution (pH 8.0), 150 units of cholesterol oxidase, 100 mg of $NO_2$—TB and 100 mg of GSH are dissolved and the whole volume is made 1000 ml.

(3) Color producing reagent solution G

To the color producing reagent solution F, 6000 units of peroxidase is added and the whole volume is made 1000 ml.

(4) Color producing reagent solution H

To the color producing reagent solution G, 1 g of phenol is added and the whole volume is made 1000 ml.

*Experimental Method*

To 50 µl of the cholesterol solution, each 4 ml of the color producing reagent solutions F, G, and H is added and mixed, followed by incubation in a constant temperature chamber at 37°C for a predetermined time (10 minutes). Then, absorbances at wavelength of 560 nm are measured using the reagent blank as control. The results are shown in Fig. 3, wherein changes of absorbances with the lapse of time are plotted.

As is clear from Fig. 3, the addition of both peroxidase and phenol makes the reaction faster and the maximum coloring can be obtained after 4 to 6 minutes. Such an effect can be admitted when only peroxidase is added.

Experiment 3

Since the reaction is accelerated by the addition of peroxidase and phenol and the coloring is stable, calibration curves are obtained by changing the cholesterol concentration. Further, the amount of $NO_2$—TB in reagents solutions is changed and compared.

*Reagents*

(1) Standard solutions

Standard solutions are prepared by dissolving 40 mg, 80 mg, 120 mg, 160 mg and 200 mg of cholesterol in isopropanol and making the volume 100 ml, respectively.

(2) Color producing reagent solution I

In 0.1 M tris buffer solution (pH 8.0), 150 units of cholesterol oxidase, 100 mg of GSH, 6000 units of peroxidase, 1 g of phenol, and 100 mg of $NO_2$—TB are dissolved and the whole volume is made 1000 ml.

(3) Color producing reagent solution J

The amount of $NO_2$—TB in the color producing reagent solution I is changed to 200 mg from 100 mg.

*Experimental Method*

To each 50 µl of the standard solutions, each 4 ml of the color producing reagent solutions I and J is added, followed by incubation in a constant temperature chamber at 37°C for 10 minutes. Then, absorbances at wavelength of 560 nm are measure using reagent blank as control.

The results are shown in Table 2. Fig. 4 shows calibration curves.

As is clear from Fig. 4, linearity is shown at the $NO_2$—TB concentrations of 10 mg/dl and 20 mg/dl.

TABLE 2

| Cholesterol concentration \ $NO_2$—TB concentration | 10 mg/dl | 20 mg/dl |
|---|---|---|
| 40 mg/dl | 0.143 | 0.147 |
| 80 | 0.280 | 0.298 |
| 120 | 0.446 | 0.459 |
| 160 | 0.582 | 0.596 |
| 200 | 0.721 | 0.741 |
| Reagent blank | 0.013 | 0.021 |

Experiment 4

Absorption Curve and Sensitivity

*Reagents*

(1) Cholesterol solution

The same as in Experiment 1.

(2) Color producing reagent solution K

In 0.1 M tris buffer solution (pH 8.0), 150 units of cholesterol oxidase, 100 mg of GSH, 6000 units of peroxidase, 1 g of phenol and 200 mg of $NO_2$—TB are dissolved and the whole volume is made 1000 ml.

*Experimental Method*

To 50 μl of the cholesterol solution, 4 ml of the color producing reagent solution is added and mixed, followed by incubation in a constant temperature chamber at 37°C for 10 minutes. Then, absorbance at wavelength of 400 to 700 nm is measured.

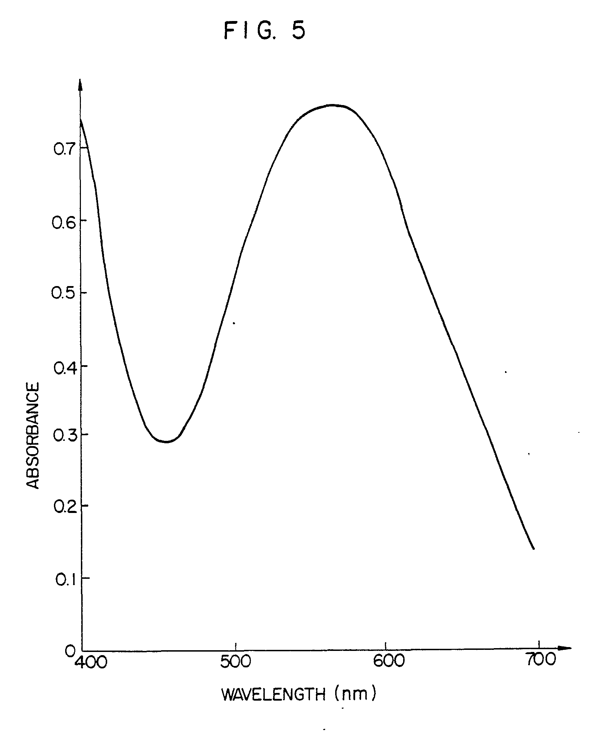

The absorption curve is shown in Fig. 5. As is clear from Fig. 5, the sensitivity of superoxide ion is high.

Example 1

Quantitative determination of free cholesterol in blood serum

*Reagents*

(1) Standard solutions

Standard solutions are prepared by dissolving 40 mg, 80 mg, 120 mg, 160 mg, and 200 mg of cholesterol in isopropanol and making the volume 100 ml, respectively.

(2) Color producing reagent solution

In 0.1 M tris buffer solution (pH 8.0), 150 units of cholesterol oxidase, 100 mg of GSH, 6000 units of peroxidase, 1 g of phenol, 20 mg of potassium cyanide and 200 mg of $NO_2$—TB are dissolved and the whole volume is made 1000 ml.

*Measuring Method*

To 50 μl of blood serum, 4.0 ml of the color producing reagent solution is added and incubated in a constant temperature chamber at 37°C for 10 minutes. Absorbance at wavelength of 560 nm is measured by using reagent blank as control.

On the hand, using the standard solutions containing cholesterol in amounts 40, 80, 120, 160 and 200 mg/dl, the calibration curve is prepared from absorbances measured in the same manner as mentioned above. The content of free cholesterol in blood serum is determined using said calibration curve.

The results are shown in Table 3.

TABLE 3

| Serum No. | Amount (mg/dl) |
|---|---|
| 1 | 44.7 |
| 2 | 51.0 |
| 3 | 66.2 |
| 4 | 49.3 |
| 5 | 36.8 |
| Average | 49.6 |

The above-mentioned process can be applied to other substrates such as glucose, glycerol, glycerol phosphate, choline, acyl CoA, pyruvic acid, uric acid, xanthine and lactic acid. In such cases, individual specific oxidases corresponding to substrates to be determined are used in place of cholesterol oxidase in the case of determination of cholesterol.

As mentioned above, the superoxide ion can produce color by reducing a reagent to be reduced. To measure the degree of coloring or the degree of color change is very easy considering today's spectroscopic technique. It is one advantage of this invention to remove influences of bilirubin, ascorbic acid, etc., having slight reducing power present in body fluid by properly selecting a color reducing reagent to be reduced. Further, it is another advantage of this invention to select freely a color producing reagent to be reduced which has high sensitivity and can produce color at longer wavelength side.

The superoxide ion changes to hydrogen peroxide, although there may a difference in speed, and it has been believed that said change can be accelerated by superoxide dismutase usually present in serum (the amount in the serum is very minute, unless hemolysis takes place). Another important thing in this invention is that the course to hydrogen peroxide previously taken is replaced completely by the course to reduction by superoxide. This can be done by the use of a compound having a SH group, that is, a thiol compound. The objected effect of thiol compound can be helped and accelerated by the addition of a phenol (including a naphthol, hereinafter the term "phenol" includes naphthols). It is also admitted that the

co-use of a peroxidase makes the quantitativeness of the process of this invention sufficiently satisfactory.

Thus, a substrate to be measured is treated with an oxidase (a) which has a specificity to said substrate, and the superoxide ion generated quantitatively from the enzymic reaction is measured by applying its reducing properties to quantitatively determine the substrate. In such a case, (b) a peroxidase, (c) an amine and/or a phenol, (d) a thiol compound having a SH group and (e) a color producing reagent to be reduced are used for improving the measuring time, sensitivity, quantitativeness and the like, which properties are necessary for practical application of this invention.

Further, in order to remove autooxidation which is an undesirable side reaction at the time of measurement and probably caused by the thiol compound and the like additives, the addition of a chelating agent is preferable in order to proceed the desired reaction stably. That is, a reagent composition comprising (a) an oxidase, (b) a peroxidase, (c) an amine and/or a phenol, (d) a thiol compound, (e) a color producing reagent to be reduced and (f) a chelating agent is used for such a purpose.

For example, to 0.1 mole of tris buffer solution (pH 8.0), there are dissolved $2.5 \times 10^{-5}$ mole of cytochrome C, 15 units/dl of cholesterol oxidase, 600 units/dl of peroxidase, and 0.1% by weight of phenol. To the resulting solution, a solution obtained by dissolving 0.8% by weight of glutathione (reduced form) in the same buffer solution as mentioned above is added. To the resulting mixture, isopropanol containing 200 mg/dl of cholesterol is added and incubated at 37°C for, e.g. 10 minutes. Cytochrome C forms color and absorbance at a wavelength of 550 nm using the reagent blank as control (i.e., O.D. (—Bl)) is 0.10. When only isopropanol is used in place of the isopropanol solution containing cholesterol, there is no color formation of cytochrome C. Further, the same results are obtained when 2,2' - di(4 - nitrophenyl) - 5,5' - diphenyl - 3,3' - (3,3' - dimethoxy - 4,4' - diphenylene)ditetrazolium chloride (hereinafter referred to as "$NO_2$—TB") is used as color producing reagent to be reduced. These color formation are found to be damaged by the presence of a large amount of superoxide dismutase which functions specifically to superoxide ion. That is, the color formation used in this invention is admitted to be caused by the reducing action of superoxide ion.

Oxidases are oxidizing enzymes and there are specific oxidases corresponding to individual substrates. Substrates and corresponding specific oxidases usable in this invention can be listed as follows:

| | |
|---|---|
| Glucose | Glucose oxidase |
| Cholesterol | Cholesterol oxidase |
| Glycerol | Glycerol oxidase |
| Glycerolphosphate | Glycerolphosphate oxidase |
| Choline | Choline oxidase |
| Acyl CoA | Acyl CoA oxidase |
| Pyruvic acid | Pyruvate oxidase |
| Uric acid | Uricase |
| Xanthine | Xanthine oxidase |
| Lactic acid | Lactate oxidase |

These oxidases can be obtained from living bodies producing these oxidases and can be available commercially as well as peroxidases.

As the amine, there can be used conventional organic amines. Aromatic amines are more effective than aliphatic amines with a small using amount. There can be used primary amines, secondary amines and tertiary amines. Examples of these amines are aniline, N-ethylaniline, N,N-dimethylaniline, N,N-diethylaniline, N,N-diethyl-m-toluidine, N-ethyl-N-β-hydroxyethyl-m-toluidine and the like. The amine can be used in an amount of 0.0001% to 0.2% by weight in the reaction solution at the stage of color formation.

The phenol is not particularly influenced by other substituents. As the phenol compound, there can be used phenol, a chlorophenol, a dichlorophenol, a naphthol, or a sulfonic acid derivative and the like. The phenol can be used in an amount of 0.0001% to 0.2% by weight in the reaction solution at the stage of color formation.

A phenol and an amine can be used together. Further, there can be used a compound which belongs to phenols and also to amines, for example, 1-N,N-dimethylamino-4-naphthol, or 4-N,N-diethylaminosalicylic acid, or the like. But in the case of an amine, L-amino acid, or the like substrate and amine oxidase, L-amino acid oxidase, or the like oxidase, the use of phenol, not amine, is, needless to say, preferable.

As the thiol compound, there can be used reduced glutathione, thioglycolic acid, mercaptoethanol,

thiosalicylic acid, cysteamine, cysteine, or dimercaptosuccinic acid, for example. The thiol compound can be used in an amount of 1 to 50 mg/dl in the reaction solution of the stage of color formation.

The color producing reagent to be reduced means a reagent which has a suitable oxidation reduction potential and produces color by the reduction with superoxide ion. Examples of the color producing reagent to be reduced are tetrazolium salts such as $NO_2$—TB, 2 - (4 - iodophenyl) - 3 - (4 - nitrophenyl) - 5 - phenyltetrazolium chloride (hereinafter referred to as "INT"), 3 - (4,5 - dimethylthiazolyl - 2 - ) - 2,5 - diphenyltetrazolium bromide (hereinafter referred to as "MTT"), etc.; cytochrome C, tetranitromethane (very dangerous), plastocyanin, or Blue protein, for example. The color producing reagent to be reduced can be used in an amount of 1 to 40 mg/dl in the reaction solution at the stage of color formation. The tetrazolium compounds had various problems in that formazans produced by reduction of tetrazolium compounds are difficultly soluble in water, quantitativeness of color formation is not good, and devices are contaminated, and the like, but recent development obtained by introducing a group which improves the solubility into tetrazolium compounds solved such problems and makes it possible to employ these compounds in this invention.

As the chelating agent, there can be used ethylenediaminetetraacetic acid (EDTA), transcyclohexanediaminetetraacetic acid or trans-1,2-cyclohexanediamine-N,N,N',N'-tetraacetic acid (CyDTA), diethylenetriaminepentaacetic acid or diethylenetriamine-N,N,N',N'-pentaacetic acid (DTPA), etc. The chelating agent can be used in an amount of 0.5 to 5 mM/dl in the reaction solution at the stage of color formation. The addition of the chelating agent makes the variation of reagent blank values small.

The peroxidase can be used in an amount of 50 to 1000 units/dl in the reaction solution at the stage of color formation.

In the case of using the above-mentioned compounds in proper combination, if the final mixture to be measured on its coloring is clouded so as to damage the measurement, a surface active agent or solubility aid can be added thereto according to a conventional process.

The presence of anticoagulants such as heparin, sodium citrate, sodium oxalate, etc., and glycolytic inhibitors such as sodium fluoride, etc., do not influence the color formation according to the process and reagents of this invention. Further, the presence of ascorbic acid, bilirubin, hemoglobin, uric acid, pyruvic acid, glucose and the like which are present in a living body physiologically, or pathologically, or by the dosage for treatment, do not influence the color formation according to the process and reagents of this invention because of specificity of individual oxidases for objected substrates.

In practical measurement, to a sample to be tested, a mixture of (a) a special oxidase for a substrate to be determined, (b) peroxidase, (c) an amine and/or a phenol, (d) a thiol compound, and (e) a color producing reagent to be reduced, and if necessary for better results, (f) a chelating agent is added in a suitable medium (usually in a buffer solution) and incubated so as to proceed the desired reaction to a desired degree, and the resulting color formation or color change is measured to quantitatively determine the substrate content in the sample. For such a purpose, the oxidase and the like additives and reagents are mixed into one or into several groups or can be used alone. Various combinations of the above-mentioned additives and reagents, along or as a mixture thereof, are possible for providing the reagents used in the process of this invention. The medium for the reagents or the reaction solution is preferably made pH 7.0 or more, more preferably 7.5 or more, during this determination.

As mentioned above, this invention provides a process and a mixture of reagents for quantitative determination of a substrate in body fluid component wherein superoxide ion is measured. Such an invention is epock-making and contributes to this field of art greatly.

This invention is illustrated by way of the following Examples.

Example 2 (cholesterol)

A color producing reagent solution is prepared by dissolving in 0.1 M tris buffer solution (pH 8.0) 20 mg/dl of $NO_2$—TB, a phenol or amine compound as listed in Table 4 in an amount of 1.06 mM/l., 0.65 mM/l. reduced glutathione, 300 U/dl of peroxidase, 15 U/dl of cholesterol oxidase, 0.1 g/dl of Triton X—100 (octylphenoxypolyethoxyethanol-available from Rohm and Haas Co.) and 0.4 g/dl of Emaglen 920 (available from Kao-Atlas Co., Ltd.). On the other hand, a sample solution to be tested is prepared by dissolving 200 mg of cholesterol in isopropanol and making the volume 100 ml.

To 50 μl of the sample solution, 3.0 ml of the color producing solution is added and incubated in a constant temperature chamber at 37°C for 10 minutes. Absorbance at wavelength of 560 nm is measured using each reagent blank as control.

The results are as shown in Table 4. As is clear from Table 4, the effect of addition of phenols or amines is remarkable.

TABLE 4

| Amine or Phenol | O.D. (—Bl) |
|---|---|
| No addition | 0.39 |
| Phenol | 1.27 |
| p-Chlorophenol | 1.24 |
| o-Chlorophenol | 1.25 |
| m-Chlorophenol | 1.23 |
| 2,4,6-Trichlorophenol | 1.24 |
| Aniline | 1.26 |
| N-Ethylaniline | 1.27 |
| N,N-Diethylaniline | 1.26 |
| N,N-Dimethyl-m-toluidine | 1.26 |
| N-Ethyl-N-β-hydroxyethyl-m-toluidine | 1.27 |
| 4-Diethylaminosalicylic acid | 1.15 |
| 1-Dimethylaminonaphthalene-7-sulfonic acid | 1.22 |
| 1-Naphthol-8-sulfonic acid | 1.04 |

Example 3 (Cholesterol)

A color producing reagent solution is prepared by dissolving in 0.1 M tris buffer slution (pH 8.0) 20 mg/dl of $NO_2$—TB, 1.06 mM/l. of phenol, 0.65 mM/l. of thiol compound as listed in Table 5, 300 U/dl. of peroxidase, and 15 U/dl of cholesterol oxidase. On the other hand, a sample solution to be tested in prepared by dissolving 200 mg of cholesterol in isopropanol and making the volume 100 ml.

To 50 μl of the sample solution, 3.0 ml of the color producing reagent solution is added and measured in the same manner as described in Example 2. The results are shown in Table 5. As is clear from Table 5, the effect of addition of thiol compound is remarkable.

TABLE 5

| Thiol compound | O.D. (—Bl) |
|---|---|
| No addition | 0.03 |
| Glutathione (reduced form) | 1.27 |
| Thioglycolic acid | 1.26 |
| L-Cysteine | 2.24 |
| Cysteamine | 1.57 |
| Thiosalicylic acid | 1.25 |
| Dimercaptosuccinic acid | 1.03 |

Example 4 (Cholesterol)

A first color producing reagent solution is prepared by dissolving in 0.1 M tris buffer solution (pH 8.0) 10 mg/dl of $NO_2$—TB, 0.1% by weight of phenol, 0.13% by weight of Triton X—100, 600 U/dl of peroxidase (Biozyme Co.), 15 U/dl of cholesterol oxidase (available from Amano Pharmaceutical Co., Ltd. 2.16 U/mg), and 100 U/dl of cholesterol esterase (available from Amano Pharmaceutical Co., Ltd., 29.2 U/mg). A second

color producing reagent solution is prepared by dissolving in 0.1 M tris buffer solution (pH 8.0) 800 mg/dl of glutathione (reduced form).

Sample A is distilled water as blank, Sample B is an isopropanol solution containing 200 mg/dl of cholesterol and Sample C is human blood serum containing 289 mg/dl of cholesterol, said value being obtained by a conventional measuring method.

The first and second color producing reagent solutions are incubated at 37°C. Three mixed solutions are prepared by mixing 4 ml of the first color producing reagent solution with 100 µl of the second color producing reagent solution. After 15 seconds, each 20 µl of Samples A, B and C is added to each resulting mixture and incubated at 37°C for 10 minutes. By measuring the absorbance at 560 nm ($O.D._{560}$), the content in Sample C is calculated as 285 mg/dl using the results shown in Table 6.

TABLE 6

| $O.D._{560}$ | | | O.D. (—Bl) | |
|---|---|---|---|---|
| Sample A | Sample B | Sample C | B—A | C—A |
| 0.103 | 0.497 | 0.665 | 0.394 | 0.562 |

When 18 samples, the cholesterol content of which are determined as 155 to 285 mg/dl according to a conventional method, are quantitatively determined using the reagents of Example 4, there are obtained the correlation coefficient of 0.923 and regression line $y = 1.002 \times -14.5$.

When 2.5 mM/l. of EDTA is added to the first color producing reagent solution and the second color producing reagent is added to the resulting mixture, the change of reagent blank value Δ is 0.002/min. On the other hand, when no EDTA is added, the change of reagent blank Δ is 0.010/min.

Example 5 (Acyl CoA)

A color producing reagent solution is prepared by dissolving in 0.1 M tris buffer solution (pH 8.0) 10 mg/dl of $NO_2$—TB, 0.1% by weight of phenol, 600 U/dl of peroxidase (derived from horse radish, available from Biozyme Co.), 20 mg/dl of glutathione (reduced form), and 240 U/dl of acyl CoA oxidase (available from Toyo Jozo Kabushiki Kaisha).

Sample A is distilled water as blank, and Sample B is a 2 mM aqueous solution of CoA (available from Toyo Jozo Kabushiki Kaisha, content 93.6%).

Each 3 ml of the color producing reagent solution is placed in two test tubes and each 100 µl of Sample A or B is added and incubated at 37°C for 10 minutes. Absorbance of Sample B is measured at wavelength of 560 nm using Sample A as control. Sample B is diluted with distilled water 2 and 4 times to give Samples B′ and B″, which are measured in the same manner as described above. The results are as shown in Table 7. As is clear from Table 7, there is obtained a linear calibration curve.

TABLE 7

| Sample | B | B′ | B″ |
|---|---|---|---|
| O.D. (—Bl) | 0.994 | 0.500 | 0.247 |

Example 6 (Glucose)

A color producing reagent solution is prepared by dissolving in 0.1 M phosphate buffer solution (pH 8.0) 10 mg/dl of INT, 0.1% by weight of 3,5-dimethoxy-N-ethyl-N-(2-hydroxy-3-sodium sulfopropyl)aniline, 600 U/dl of peroxidase (available from Biozyme Co.), 20 mg/dl of glutathione (reduced form), and 3000 U/dl of glucose oxidase (available from Amano Pharmaceutical Co., Ltd.).

Sample A is distilled water as blank, Sample B is an aqueous solution containing 200 mg/dl of glucose, and Sample C is human blood serum containing 121 mg/dl of glucose, said value being obtained by a conventional measuring method. Further, Sample C is diluted with distilled water 2 and 4 times to give Samples C′ and C″, respectively.

Each 3 ml of the color producing reagent solution is placed in 5 test tubes and each 20 µl of Samples A, B, C, C′ and C″ is added to each test tube, followed by incubation at 37°C for 10 minutes. Immediately, absorbances of Samples B to C″ are measured at wavelength of 500 nm using Sample A as control. The results are shown in Table 8.

TABLE 8

| Sample | A | B | C | C′ | C″ |
|---|---|---|---|---|---|
| $O.D._{560}$ | 0.104 | 1.104 | 0.705 | 0.410 | 0.250 |
| O.D. (—Bl) | — | 1.000 | 0.601 | 0.306 | 0.146 |

From the above results, the content of glucose in Sample C is calculated as 120 mg/dl. There is admitted linearity of the calibration curve from the data of Samples C, C′ and C″.

Example 7 (Pyruvic acid)

A color producing reagent solution is prepared by dissolving in 0.02 M phosphate buffer solution (pH 7.1) 20 mg/dl of $NO_2$—TB, 0.1% by weight of phenol, 600 U/dl of peroxidase (available from Biozyme Co.), 10 mg/dl of glutathione (reduced form), 700 U/dl of pyruvate oxidase (available from Toyo Jozo Kabushiki Kaisha), 2 mg/dl of flavin adeninedinucleotide, 44 mg/dl of thiamine pyrophosphate, and 0.15% by weight of magnesium acetate.

Sample A is distilled water as blank, and Sample B is an aqueous solution containing lithium pyruvate in an amount of 10 mg/dl as pyruvic acid.

Each 3 ml of the color producing reagent solution is placed in two test tubes and each 100 µl of Samples A and B is added to each test tube and incubated at 37°C for 15 minutes. Immediately, absorption at wavelength of 560 nm of Sample B is measured using Sample A as control to give the value of 0.042.

Example 8 (Choline)

A color producing reagent solution is prepared by dissolving in 0.1 M tris buffer solution (pH 8.0) 10 mg/dl of $NO_2$—TB, 0.1% by weight of phenol, 600 U/dl of peroxidase (available from Biozyme Co.), 10 mg/dl of thiosalicylic acid, and 500 U/dl of choline oxidase (available from Toyo Jozo (Kabushiki Kaisha).

Sample A is distilled water as blank, and Sample B is an aqueous solution containing 70 mg/dl of choline chloride. Sample B is diluted with distilled water 2 and 4 times to give Samples B′ and B″, respectively.

Each 3 ml of the color producing reagent solution is placed in 4 test tubes and each 20 µl of Samples A, B, B′ and B″ is added to each test tube, followed by incubation at 37°C for 10 minutes. Immediately, absorbances of Samples B to B″ are measured at wavelength of 560 nm using Sample A as control. The results are shown in Table 9.

TABLE 9

| Sample | B | B′ | B″ |
|---|---|---|---|
| O.D. (—Bl) | 1.100 | 0.581 | 0.281 |

Example 9 (Glycerol-3-phosphate)

A color producing reagent solution is prepared by dissolving in 0.1 M tris buffer solution (pH 8.0) 10 mg/dl of $NO_2$—TB, 0.05% by weight of phenol, 600 U/dl of peroxidase (available from Biozyme Co.), 20 mg/dl of glutathione (reduced form), and 600 U/dl of glycerol-3-phosphate oxidase (available from Toyo Jozo Kabushiki Kaisha).

Sample A is distilled water as blank and Sample B is an aqueous solution containing 10 mM (172 mg/dl) of glycerol-3-phosphate. Sample B is diluted with distilled water 2 and 4 times to give Samples B′ and B″, respectively.

Each 4 ml of the color producing reagent solution is placed in 4 test tubes and each 50 µl of Samples A, B, B′ and B″ is added to each test tube, followed by incubation at 37°C for 10 minutes. Immediately, absorbances of Samples B to B″ are measured at wavelength of 560 nm using Sample A as control. The results are shown in Table 10.

TABLE 10

| Sample | B | B′ | B″ |
|---|---|---|---|
| O.D. (—Bl) | 0.678 | 0.344 | 0.169 |

Example 10 (Glycerol)

A color producing reagent solution is prepared by dissolving in 0.05 M phosphate buffer solution 10

mg/dl of $NO_2$—TB, 0.05% by weight of phenol, 600 U/dl of peroxidase (available from Biozyme Co.), 20 mg/dl of glutathione (reduced form), and 600 U/dl of glycerol oxidase (available from Kyowa Hakko Kogyo Co., Ltd.).

Sample A is distilled water as blank, and Sample B is an aqueous solution containing 2 mM of glycerin. Sample B is diluted with distilled water 2 and 4 timers to give Samples B' and B", respectively.

Each 4 ml of the color producing reagent solution is placed in 4 test tubes and each 50 μl of Samples A, B, B' and B" is added to each test tube, followed by incubation at 37°C for 10 minutes. Immediately absorbances of Samples B to B" are measured at wavelength of 560 nm using Sample A as control. The results are shown in Table 11.

TABLE 11

| Sample | B | B' | B" |
|---|---|---|---|
| O.D. (—Bl) | 0.081 | 0.038 | 0.020 |

Example 11 (Uric acid)

A color producing reagent solution is prepared by dissolviing in 0.1 M tris buffer solution (pH 7.1) 20 mg/dl of $NO_2$—BT, 0.1% by weight of phenol, 600 U/dl of peroxidase (available from Biozyme Co.), 10 mg/dl of glutathione (reduced form), and 30 U/dl of uricase (available from Toyobo Co., Ltd.).

Sample A is distilled water as blank, Sample B is a solution containing 10 mg/dl of uric acid obtained by dissolving 10 mg of uric acid in 100 ml of a 1% aqueous solution of lithium carbonate, and Sample C is high uric acid content serum containing 12 mg/dl of uric acid, said value being obtained by a conventional measuring method.

Each 3 ml of the color producing reagent solution is placed in 3 test tubes and each 60 μl of Samples A, B and C is added to each test tube, followed by incubation at 37°C for 10 minutes. Immediately, absorbances of Samples B and C are measured at wavelength of 560 nm using Sample A as control. From the results shown in Table 12, the content of uric acid in Sample C is calculated as 21.8 mg/dl.

TABLE 12

| Sample | B | C |
|---|---|---|
| Absorbance | 0.074 | 0.088 |

Example 12 (L-Lactic acid)

A color producing reagent solution is prepared by dissolving in 0.1 M tris buffer solution (pH 7.5) 20 mg/dl of $NO_2$-TB, 0.1% by weight of phenol, 600 U/dl of peroxidase (available from Biozyme Co.), 10 mg/dl of glutathione (reduced form), and 85 U/dl of L-lactate oxidase.

Sample A is distilled water as blank, and Sample B is an aqueous solution containing 10 mM of sodium L-lactate and making the volume 100 ml.

Each 3 ml of the color producing reagent solution is placed in two test tubes and each 60 μl of Samples A and B is added to each test tube, followed by incubation at 37°C for 10 minutes. Immediately, absorbance of Sample B is measured at wavelength of 560 nm using Sample A as control to give the value of 0.110.

Example 13 (Cholesterol)

A first color producing reagent solution is prepared by dissolving in 0.1 M tris buffer solution (pH 8.0) $3 \times 10^{-5}$ mole of oxidizing type cytochrome C (available from Sigma Chemical Co., type III), 0.1% by weight of phenol, 600 U/dl of peroxidase (available from Biozyme Co.), and 15 U/dl of cholesterol oxidase (available from Amano Pharmaceutical Co., Ltd.). A second color producing reagent solution is prepared by dissolving in 0.1 M tris buffer solution (pH 8.0) 800 mg/dl of glutathione (reduced form).

Sample A is isopropanol as blank, Sample B is an isopropanol solution containing 200 mg/dl of cholesterol, and Sample C is blood serum containing 230 mg/dl of cholesterol, said value being obtained by a conventional measuring method. Further, Sample C is diluted with isopropanol 2 and 4 times to give Samples C' and C'', respectively.

The first and second color producing reagent solutions are preheated at 37°C. Immediately before the measurement, to 4 ml of the first color producing reagent solution, 100 μl of the second color producing reagent solution and 20 μl of a sample are added and incubated at 37°C for accurately 2 minutes. Subsequently, absorbances at wavelengh of 550 nm are measured. The results are shown in Table 13.

TABLE 13

| Sample | A | B | C | C' | C'' |
|---|---|---|---|---|---|
| O.D.$_{550}$ | 0.210 | 0.316 | 0.336 | 0.274 | 0.240 |
| O.D. (−B1) | — | 0.106 | 0.126 | 0.064 | 0.030 |

From the above results, the content of cholesterol in Sample C is 238 mg/dl. The values of Samples C, C' and C'' show linearity.

In the above-mentioned process, peroxidase is used, but the same effects can be obtained by using iron complex of porphyrin or iron chelate of complexanes in place of peroxidase.

That is, a substrate to be measured is treated with an oxidase (a) which has a specificity to said substrate, and the generated superoxide ion quantitatively from the enzymic reaction is measured by applying its reducing properties to quantitatively determine the substrate. In such a case, (b') iron complex of porphyrin or iron chelate of complexane, (c) an amine and/or a phenol, (d) a thiol compound and (e) a color producing reagent to be reduced are used for improving the measuring time, sensitivity, quantitativeness and the like, which properties are necessary for practical application of this invention. Thus, the reaction rates of the enzymic reaction of oxidase (i.e. the formation of superoxide ion) and the reducing reaction of the superoxide ion against the color producing reagent to be reduced can be accelerated effectively, quantitative determination of superoxide ion (or in other words, quantitative determination of substrate) which is generated by treating a substrate with an oxidase can be improved in quantitativeness, and requirements for the measuring time and sensitivity can be satisfied sufficiently.

Further, in order to remove autooxidation which is an undesirable side reaction at the time of measurement and probably caused by the thiol compound and the like additives, the addition of a chelating agent is preferable in order to proceed the desired reaction stably. That is, a reagent composition comprising (a) an oxidase, (b') iron complex of porphyrin or iron chelate of complexane, (c) and amine and/or a phenol, (d) a thiol compound, (e) a color producing reagent to be reduced and (f) a chelating agent is used for such a purpose.

The process of this invention can be carried out as follows.

For example, in 0.1 mole (M) of tris (precisely tris-HCl) buffer solution (pH 8.0), there are dissolved $2.5 \times 10^{-5}$ mole of cytochrome C, 15 units/dl of cholesterol oxidase, 1 mg/dl of hemin, and 0.1% by weight of phenol. To the resulting solution, a solution obtained by dissolving 0.8% by weight of glutathione (reduced form) in the same buffer solution as mentioned above is added. To the resulting mixture, isopropanol containing 200 mg/dl of cholesterol is added and incubated at 37°C for, e.g. 10 minutes. Cytochrome C forms color and absorbance at a wavelength of 550 nm using the reagent blank as control (i.e., O.D. (−B1)) is 0.10. When only isopropanol is used in place of the isopropanol solution containing cholesterol, there is no color formation of cytochrome C. Further, the same results are obtained when 2,2'-di(4-nitrophenyl)-5,5'-diphenyl-3,3'-(3,3'-dimethoxy-4,4'-diphenylene)ditetrazolium chloride (hereinafter referred to as "$NO_2$—TB") is used as color producing reagent to be reduced. These color formations are found to be damaged by the presence of a large amount of superoxide dismutase which functions specifically to superoxide ion. That is, the color formation used in this invention is admitted to be caused by the reducing action of superoxide ion.

As the iron complex of porphyrin, there can be used hemin, α,β,γ,δ-tetraphenylporphyrintrisulfonic acid-iron complex, α,β,γ,δ-tetrakis(4-N-methylpyridyl)porphyrin-iron complex, tetraphenylporphyrin-iron complex, octaethylphorphyrin-iron complex, etc. The iron complex of porphyrin can be used in an amount of 0.007 to 0.06 mM/l. in the reaction solution at the stage of color formation.

As the iron chelate of complexane, there can be used iron chelates of complexanes such as ethylenediaminetetraacetic acid (EDTA), diaminopropanetetraacetic acid, trans-cyclohexanediaminetetraacetic acid, hydroxyethylethylenediaminetriacetic acid, glycol ether diaminetetraacetic acid, etc. Among them, the use of EDTA·Fe(III) is particularly preferable. The iron chelate of complexane can be used in an amount of 0.01 to 0.07 mM/l. in the reaction solution at the stage of color formation.

As to the amines, the phenols, the thiol compounds, the color producing reagents to be reduced, and the chelating agents, those mentioned previously can be used.

In the case of using the above-mentioned compounds in proper combination, if the final mixture to be measured on its coloring is clouded so as to damage the measurement, a surface active agent or solubility aid can be added thereto according to a conventional process.

The presence of anticoagulants such as heparin, sodium citrate, sodium oxalate, etc., and glycolytic inhibitors such as sodium fluoride, etc., do not influence the color formation according to the process and reagents of this invention. Further, the presence of ascorbic acid, bilirubin, hemoglobin, uric acid, pyruvic acid, glucose and the like which are present in a living body physiologically, or pathologically, or by the dosage for treatment, do not influence the color formation according to the process and reagents of this invention because of specificity of individual oxidases for objected substrates.

In practical measurement, to a sample to be tested, a mixture of (a) a special oxidase for a substrate to

be determined, (b') iron complex of porphyrin or iron chelate of complexane, (c) an amine and/or a phenol, (d) a thiol compound, and (e) a color producing reagent to be reduced, and if necessary for better results, (f) a chelating agent is added in a suitable medium (usually in a buffer solution) and incubated so as to proceed the desired reaction to a desired degree, and the resulting color formation or color change is measured to quantitatively determine the substrate content in the sample. For such a purpose, the oxidase and the like additives and reagents are mixed into one or into several groups or can be used alone. Various combinations of the above-mentioned additives and reagents, alone or as a mixture thereof, are possible for providing the reagents used in the process of this invention. The medium for the reagents or the reaction solution is preferably made pH 7.0 or higher, more preferably 7.5 or higher, during the determination.

As mentioned above, this invention provides a process and a mixture of reagents for quantitative determination of a substrate in body fluid component wherein superoxide ion is measured. Such an invention is epock-making and contributes to this field of art greatly.

The following Examples further illustrate this invention.

Example 14 (Cholesterol)

A color producing reagent solution is prepared by dissolving in 0.1 M tris buffer solution (pH 8.0) 20 mg/dl of $NO_2$—TB, 1.06 mM/l of phenol, 0.65 mM/l of reduced glutathione, 1 mg/dl of hemin, 15 U/dl of cholesterol oxidase and 0.1 g/dl of Triton X-100 (octylphenoxypolyethoxyethanol — available from Rohm and Haas Co.). On the other hand, a sample solution to be tested is prepared by dissolving 200 mg of cholesterol in isopropanol and making the volume 100 ml.

To 50 µl of the sample solution, 3.0 ml of the color producing reagent solution is added and incubated in a constant temperature chamber at 37°C for 10 minutes. Then, absorbance at wavelength of 560 nm is measured using reagent blank as control.

Reference Example 1 (Cholesterol)

In the color producing reagent solution used in Example 14, 300 U/dl of peroxidase is used in place of hemin. Absorbance is measured in the same manner as described in Example 14 using the resulting color producing reagent solution.

Comparative Example 1 (Cholesterol)

In the color producing reagent solution used in Example 14, hemin is not added. Using the resulting color producing reagent solution, absorbance is measured in the same manner as described in Example 14.

The results are shown in Table 14. As is clear from Table 14, hemin has the same effect or higher effect compared with peroxidase.

TABLE 14

| Example No. | Example 14 | Reference Example 1 | Comparative Example 1 |
|---|---|---|---|
| Absorbance | 1.391 | 1.286 | 0.264 |

Example 15 (Cholesterol)

In the color producing reagent solution used in Example 14, 1 mg/dl of EDTA·Fe(III) is used in place of hemin. Using the resulting color producing reagent solution, absorbance is measured in the same manner as described in Example 14.

The results are shown in Table 15. As is clear from Table 15, EDTA·Fe(III) has the same effect as peroxidase.

TABLE 15

| Example No. | Example 15 | Reference Example 1 | Comparative Example 1 |
|---|---|---|---|
| Absorbance | 1.264 | 1.286 | 0.264 |

Comparative Example 2 (Cholesterol)

In the color producing reagent solution used in Example 14, 1 mg/dl of 2 Na-EDTA is used in place of hemin. Using the resulting color producing reagent solution, absorbance is measured in the same manner as described in Example 14.

The results are shown in Table 16. As is clear from Table 16, no effect is obtained by 2 Na-EDTA.

TABLE 16

| Example No. | Comparative Example 2 | Example 15 | Comparative Example 1 |
|---|---|---|---|
| Absorbance | 0.258 | 1.264 | 0.264 |

Comparative Example 3 (Cholesterol)

In the color producing reagent solution used in Example 14, 1 mg/dl of EDTA·Ni(II) or EDTA·Mn(II) is used in place of hemin. Using the resulting color producing reagent solutions, absorbances are measured in the same manner as described in Example 14.

The results are shown in Table 17. As is clear from Table 17, almost no effect is admitted compared with Example 15.

TABLE 17

| Example No. | Comparative Example 3 EDTA·Ni(II) | Comparative Example 3 EDTA·Mn(II) | Example 15 | Comparative Example 1 |
|---|---|---|---|---|
| Absorbance | 0.273 | 0.275 | 1.264 | 0.264 |

Example 16 (Cholesterol)

In the color producing reagent solution used in Example 14, 1 mg/dl of diaminopropanetetraacetic acid-iron(III) (Methyl-EDTA·Fe(III)), trans-cyclohexanediaminetetraacetic acid-iron(III) (CyDTA·Fe(III)), or hydroxyethylethylenediaminetracetic acid-iron(III) (EDTA-OH·Fe(III)) is used in place of hemin. Using the resulting color producing reagent solutions, absorbances are measured in the same manner as described in Example 14.

The results are shown in Table 18. As is clear from Table 18, effects of addition of iron chelates are remarkable compared with Comparative Example 1.

TABLE 18

| Example No. | Absorbance |
|---|---|
| Example 4 | |
| Methyl-EDTA·Fe(III) | 0.979 |
| CyDTA·Fe(III) | 0.501 |
| EDTA-OH·Fe(III) | 0.400 |
| Comparative Example 1 | 0.264 |

Example 17 (Acyl CoA)

A color producing reagent solution is prepared by dissolving in 0.1M tris buffer solution (pH 8.0) 10 mg/dl of $NO_2$-TB, 1.06 mM/l. of phenol, 1 mg/dl of hemin, 20 mg/dl of reduced glutathione, and 240 U/dl of acryl CoA oxidase. On the other hand, a sample solution to be tested is prepared by dissolving in distilled water 2 mM of CoA and making the volume 100 ml.

To 100 µl of the sample solution, 3.0 ml of the color producing reagent solution is added and incubated in a constant temperature chamber at 37°C for 10 minutes. Then, absorbance at wavelength of 560 nm is measured using reagent blank as control.

Reference Example 2 (Acyl CoA)

In the color producing reagent solution used in Example 17, 600 U/dl of peroxidase is used in place of hemin. Using the resulting color producing reagent solution, absorbance is measured in the same manner as described in Example 17.

The results are shown in Table 19. As is clear from Table 19, hemin has the same effect or higher effect compared with peroxidase.

TABLE 19

| Example No. | Example 17 | Reference Example 2 |
|---|---|---|
| Absorbance | 1.100 | 0.997 |

Example 18 (Glucose)

A color producing reagent solution is prepared by dissolving in 0.1 M phosphate buffer solution (pH 8.0) 10 mg/dl of INT, 0.1% by weight of 3,5-dimethoxy-N-ethyl-N-(2-hydroxy-3-sodium sulfopropyl)aniline, 1 mg/dl of hemin, 20 mg/dl of reduced glutathion, and 3000 U/dl of glucose oxidase. On the other hand, a sample solution is prepared by dissolving in distilled water 200 mg/dl of glucose.

To 20 µl of the sample solution, 3.0 ml of the color producing reagent solution is added and incubated in a constant temperature chamber at 37°C for 10 minutes. Then, absorbance at wavelength of 500 nm is measured using reagent blank as control.

Reference Example 3 (Glucose)

In the color producing reagent solution used in Example 18, 600 U/dl of peroxidase is used in place of hemin. Using the resulting color producing reagent solution, absorbance is measured in the same manner as described in Example 18.

The results are shown in Table 20. As is clear from Table 20, hemin has the same effect or higher effect compared with peroxidase.

TABLE 20

| Example No. | Example 18 | Reference Example 3 |
|---|---|---|
| Absorbance | 1.095 | 1.000 |

Example 19 (Pyruvic acid)

A color producing reagent solution is prepared by dissolving in 0.02 M phosphate buffer solution (pH 7.1) 20 mg/dl of $NO_2$-TB, 1.06 mM/l of phenol, 1 mg/dl of hemin, 10 mg/dl of reduced glutathione, 700 U/dl of pyruvate oxidase, 2 mg/dl of flavin adenine dinucleotide, 44 mg/dl of thiamine pyrophosphate, and 0.15% by weight of magnesium acetate. On the other hand, a sample solution to be tested is prepared by dissolving lithium pyruvate in an amount of 10 mg/dl as pyruvic acid in distilled water.

To 100 µl of the sample solution, 3.0 ml of the color producing reagent solution is added and incubated in a constant temperature chamber at 37°C for 15 minutes. Then, absorbance at wavelength of 560 nm is measured using reagent blank as control to obtain the value of 0.050.

Example 20 (Choline)

A color producing reagent solution is prepared by dissolving in 0.1M tris buffer solution (pH 8.0) 10 mg/dl of $NO_2$-TB, 1.06 mM/l of phenol, 1 mg/dl of hemin, 10 mg/dl of thiosalicylic acid, and 500 U/dl of choline oxidase. On the other hand, a sample solution to be tested is prepared by dissolving 70 mg/dl of choline chloride in distilled water.

To 20 µl of the sample solution, 3.0 ml of the color producing reagent solution is added and incubated in a constant temperature chamber at 37°C for 10 minutes. Then, absorbance at wavelength of 560 nm is measured using reagent blank as control.

Reference Example 4 (Choline)

In the color producing reagent solution used in Example 20, 600 U/dl of peroxidase is used in place of hemin. Using the resulting color producing reagent solution, absorbance is measured in the same manner as described in Example 20.

The results are shown in Table 21. As is clear from Table 21, hemin has the same effect or higher effect compared with peroxidase.

TABLE 21

| Example No. | Example 20 | Reference Example 4 |
|---|---|---|
| Absorbance | 1.191 | 1.100 |

Example 21 (Glycerol-3-phosphate)

A color producing reagent solution is prepared by dissolving in 0.1 M tris buffer solution (pH 8.0) 10 mg/dl of $NO_2$-TB, 0.5 mM/l of phenol, 1 mg/dl of hemin, 20 mg/dl of reduced glutathione, and 600 U/dl of glycerol-3-phosphate oxidase. On the other hand, a sample solution is prepared by dissolving 10 mM (172 mg/dl) of glycerol-3-phosphate in distilled water.

To 50 µl of the sample solution, 4.0 ml of the color producing reagent solution is added and incubated in a constant temperature chamber at 37°C for 10 minutes. Then, absorbance at wavelength of 560 nm is measured using reagent blank as control.

Reference Example 5 (Glycerol-3-phosphate)

In the color producing reagent solution used in Example 21, 600 U/dl of peroxidase is used in place of hemin. Using the resulting color producing reagent solution, absorbance is measured in the same manner as described in Example 21.

The results are shown in Table 22. As is clear from Table 22, hemin has the same effect or higher effect compared with peroxidase.

TABLE 22

| Example No. | Example 21 | Reference Example 5 |
|---|---|---|
| Absorbance | 0.725 | 0.678 |

Example 22 (Glycerol)

A color producing reagent solution is prepared by dissolving in 0.05 M phosphate buffer solution (pH 8.0) 10 mg/dl of $NO_2$-TB, 0.5 mM/l of phenol, 1 mg/dl of hemin, 20 mg/dl of reduced glutathione, and 600 U/dl of glycerol oxidase. On the other hand, a sample solution is prepared by dissolving 2 mM of glycerin in distilled water and making the volume 100 ml.

To 50 µl of the sample solution, 4.0 ml of the color producing reagent solution is added and incubated in a constant temperature chamber at 37°C for 10 minutes. Then, absorbance at wavelength of 560 nm is measured using reagent blank as control.

Reference Example 6 (Glycerol)

In the color producing reagent solution used in Example 22, 600 U/dl of peroxidase is used in place of hemin. Using the resulting color producing reagent solution, absorbance is measured in the same manner as described in Example 22.

The results are shown in Table 23. As is clear from Table 23, hemin has the same or higher effect compared with peroxidase.

TABLE 23

| Example No. | Example 22 | Reference Example 6 |
|---|---|---|
| Absorbance | 0.090 | 0.081 |

Example 23 (Uric acid)

A color producing reagent solution is prepared by dissolving in 0.1 M tris buffer solution (pH 7.1) 20 mg/dl of $NO_2$-TB, 1.06 mM/l of phenol, 1 mg/dl of hemin, 10 mg/dl of reduced glutathione, and 30 U/dl of uricase. On the other hand, a sample solution to be tested is prepared by dissolving uric acid in an amount of 10 mg/dl in a 1% aqueous solution of lithium carbonate.

To 60 µl of the sample solution, 3.0 ml of the color producing reagent solution is added and incubated in a constant temperature chamber at 37°C for 10 minutes. Then, absorbance at wavelength of 560 nm is measured using reagent blank as control.

Reference Example 7 (Uric acid)

In the color producing reagent solution used in Example 23, 600 U/dl of peroxidase is used in place of hemin. Using the resulting color producing reagent solution, absorbance is measured in the same manner as described in Example 23.

The results are shown in Table 24. As is clear from Table 24, hemin has the same or higher effect compared with peroxidase.

TABLE 24

| Example No. | Example 23 | Reference Example 7 |
|---|---|---|
| Absorbance | 0.081 | 0.074 |

Example 24 (L-Lactic acid)

A color producing reagent solution is prepared by dissolving in 0.1 M tris buffer solution (pH 7.5) 20 mg/dl of $NO_2$-TB, 1.06 mM/l of phenol, 1 mg/dl of hemin, 10 mg/dl of reduced glutathione, and 85 U/dl of L-lactate oxidase. On the other hand, a sample solution is prepared by dissolving 10 mM of sodium L-lactate in distilled water and making the volume 100 ml.

To 50 µl of the sample solution, 3.0 ml of the color producing reagent solution is added and incubated in a constant temperature chamber at 37°C for 10 minutes. Then, absorbance at wavelength of 560 nm is measured using reagent blank as control.

Reference Example 8 (L-Lactic acid)

In the color producing reagent solution used in Example 24, 600 U/dl of peroxidase is used in place of hemin. Using the resulting color producing reagent solution, absorbance is measured in the same manner as described in Example 24.

The results are shown in Table 25. As is clear from Table 25, hemin has the same or higher effect compared with peroxidase.

TABLE 25

| Example No. | Example 24 | Reference Example 8 |
|---|---|---|
| Absorbance | 0.122 | 0.110 |

Example 25 (Free cholesterol in blood serum)

A color producing reagent solution is prepared by dissolving in 0.1 M tris buffer solution (pH 8.0) 20 mg/dl of $NO_2$-TB, 2 mM/l of phenyl, 0.65 mM/l of reduced glutathione, 0.017 mM/l of EDTA·Fe(III), 15 U/dl of cholesterol oxidase and 0.1 g/dl of Triton X-100.

To 50 µl of blood serum, 3.0 ml of the color producing reagent solution is added and incubated in a constant temperature chamber at 37°C for 10 minutes. Then, absorbance at wavelength of 560 nm is measured using reagent blank as control.

The cholesterol content in the sample is calculated by the calibration curve obtained previously.

Reference Example 9 (Free cholesterol in blood serum)

A color producing reagent solution is prepared by dissolving in 0.1 M phosphate buffer solution (pH 7.0) 0.1% by weight of phenol, 0.01% by weight of 4-aminoantipyrine, 10 U/dl of cholesterol oxidase, 300 U/dl of peroxidase, and 0.15% by weight of Triton X-100.

To 50 µl of blood serum, 3.0 ml of the color producing reagent solution is added and incubated in a constant temperature chamber at 37°C for 15 minutes. Then, absorbance at wavelength of 505 nm is measured using reagent blank as control.

The cholesterol content in the sample is calculated by the calibration curve obtained previously.

As shown in Table 26, the values obtained in Example 25 and Reference Example 9 are in good agreement and no significant difference is admitted.

TABLE 26

| Blood serum No. | Example 25 X | Reference Example 9 Y |
|---|---|---|
| 1 | 46 mg/dl | 47 mg/dl |
| 2 | 40 | 40 |
| 3 | 38 | 37 |
| 4 | 59 | 61 |
| 5 | 34 | 35 |
| 6 | 52 | 50 |
| 7 | 55 | 56 |
| 8 | 81 | 81 |
| 9 | 32 | 33 |
| 10 | 43 | 41 |
| Average | 48.0 | 48.1 |
| S.D. | 14.6 | 14.7 |

r = 0.996 Y = 1.00X −0.13

Example 26 (Free cholesterol in blood serum)

In the color producing reagent solution used in Example 25, 2.5 mM/l of EDTA·Fe(III) is used in place of 0.017 mM/l of EDTA·Fe(III).

To 50 µl of the sample solution used in Example 25, 3.0 ml of the above-mentioned color producing reagent solution is added and incubated in a constant temperature chamber at 37°C for 10 minutes. Then, absorbance of the sample ($E_S$) and that of reagent blank ($E_{BL}$) are measured using water as control.

The results are shown in Table 27. As is clear from Table 27, effects of addition of EDTA·Fe(III) are admitted.

TABLE 27

| | Addition of EDTA·Fe(III) | | | No addition of EDTA·Fe(III) | | |
|---|---|---|---|---|---|---|
| | $E_S$ | $E_{BL}$ | $E_S—E_{BL}$ | $E_S$ | $E_{BL}$ | $E_S—E_{BL}$ |
| Absorbance | 1.458 | 0.198 | 1.260 | 1.563 | 0.299 | 1.264 |

Example 27 (Cholesterol)

A color producing reagent solution is prepared by dissolving in 0.1 M tris buffer solution (pH 8.0) 20 mg/dl of $NO_2$-TB, 1.06 mM/l of a phenol or amine compound listed in Table 28, 0.65 mM/l of reduced glutathione, 1 mg/dl of EDTA·Fe(III), 15 U/dl of cholesterol oxidase, 0.1 g/dl of Triton X-100 and 0.4 g/dl of Emulgen 920. On the other hand, a sample solution to be tested is prepared by dissolving 200 mg of cholesterol in isopropanol and making the volume 100 ml.

To 50 µl of the sample solution, 3.0 ml of the color producing reagent solution is added and incubated in a constant temperature chamber at 37°C for 10 minutes. Then, absorbance at wavelength of 560 nm is measured using reagent blank as control.

The results are shown in Table 28. Table 28 clearly shows the effect of addition of phenols and amines.

TABLE 28

| Amine or phenol | O.D. (−B1) |
|---|---|
| No addition | 0.38 |
| Phenol | 1.26 |
| p-Chlorophenol | 1.26 |
| o-Chlorophenol | 1.26 |
| m-Chlorophenol | 1.25 |
| 2,4,6-Trichlorophenol | 1.25 |
| Aniline | 1.28 |
| N-Ethylaniline | 1.28 |
| N,N-Diethylaniline | 1.27 |
| N,N-Dimethyl-m-toluidine | 1.28 |
| N-Ethyl-N-β-hydroxyethyl-m-toluidine | 1.28 |
| 4-N,N-Diethylaminosalicylic acid | 1.20 |
| 1-N,N-Dimethylaminonaphthalene-7-sulfonic acid | 1.23 |
| 1-Naphthol-8-sulfonic acid | 1.10 |

Example 28 (Cholesterol)

A color producing reagent solution is prepared by dissolving in 0.1 M tris buffer solution (pH 8.0) 20 mg/dl of $NO_2$-TB, 1.06 mM/l of phenol, 0.65 mM/l of thiol compound as listed in Table 29, 1 mg/dl of EDTA·Fe(III), and 15 U/dl of cholesterol oxidase. On the other hand, a sample solution to be tested is prepared by dissolving 200 mg of cholesterol in isopropanol and making the volume 100 ml.

To 50 µl of the sample solution, 3.0 ml of the color producing reagent solution is added and incubated in a constant temperature chamber at 37°C for 10 minutes. Then, absorbance at wavelength of 560 nm is measured using reagent blank as control.

The results are shown in Table 29. Table 29 clearly shows the effect of addition of thiol compounds.

TABLE 29

| Thiol compound | O.D. (−B1) |
|---|---|
| No addition | 0.03 |
| Glutathione (reduced form) | 1.26 |
| Thioglycolic acid | 1.27 |
| L-Cysteine | 2.24 |
| Cysteamine | 1.59 |
| Thiosalicyclic acid | 1.26 |
| Dimercaptosuccinic acid | 1.03 |

**Claims**

1. A process for determining a substrate, said process comprising treating a sample containing said substrate with a specific oxidase corresponding to said substrate to generate superoxide ion, and reducing a colour-producing reagent with said superoxide ion, characterised in that said process is a quantitative process which involves a colorimetric measurement of the reduced colour-producing reagent and further involves treatment with a peroxidase, or an iron complex of porphyrin or an iron chelate of complexane, and a phenol and/or amine compound, and a thiol compound.

2. A process as claimed in Claim 1, wherein the substrate is glucose, cholesterol, glycerol, glycerol phosphate, choline, acyl CoA, pyruvic acid, uric acid xanthine or lactic acid.

3. A process as claimed in Claim 1 or Claim 2, wherein said sample is treated with a composition comprising (a) said oxidase, (b) said peroxidase or said iron complex of porphyrin or said iron chelate of complexane, (c) said phenol compound and/or said amine, (d) said thiol compound, (e) said colour producing reagent, and (f) a chelating agent.

4. A process as claimed in any preceding claim wherein said sample is treated with a said amine and said amine is aniline, N-ethyl-aniline, N,N-dimethylaniline, N,N-diethylaniline, N,N-diethyl-m-toluidine, N-ethyl-N-β-hydroxyethyl-m-toluidine, 1-N,N-dimethyl-amino-4-naphthol, or 4-N,N-diethylaminosalicylic acid.

5. A process as claimed in any preceding claim wherein said sample is treated with a said phenol compound and said phenol compound is phenol, a chlorophenol, a dichlorophenol, a naphthol, or a sulfonic acid derivative.

6. A process as claimed in any preceding claim wherein said thiol compound is reduced glutathione, thioglycolic acid, mercaptoethanol, thiosalicylic acid, cysteamine, cysteine, or dimercaptosuccinic acid.

7. A process as claimed in any preceding claim wherein said colour-producing reagent is a tetrazolium salt, cytochrome C, plastocyanin, or Blue protein.

8. A composition for quantitative determination of a substrate comprising (a) a specific oxidase corresponding to a substrate to be determined, (b) a peroxidase, (c) a phenol and/or an amine, (d) a thiol compound, and (e) a colour producing reagent reducible by superoxide ion.

9. A composition for quantitative determination of a substrate comprising (a) a specific oxidase corresponding to a substrate to be determined, (b') iron complex of porphyrin or iron chelate of complexane, (c) an amine and/or a phenol, (d) a thiol compound and (e) a colour producing reagent reducible by superoxide ion.

10. A composition according to Claim 8 or Claim 9, which further comprises (f) a chelating agent.

**Patentansprüche**

1. Verfahren zum Bestimmen eines Substrats, in dem eine das Substrat enthaltende Probe mit einer dem Substrat entsprechenden Oxidase behandelt und dadurch das Hyperoxidion erzeugt wird, und daß mit dem Hyperoxidion ein farberzeugendes Reagens reduziert wird, dadurch gekennzeichnet, daß das Verfahren ein quantitatives Verfahren ist, in dem eine kolorimetrische Messung des reduzierten farberezugenden Reagens vorgenommen und eine Behandlung mit einer Peroxidase oder mit einer Eisenkomplexverbindung des Porphyrins oder einem Eisenchelat des Komplexans, und mit einer Phenol- und/oder Aminverbindung und einer Thiolverbindung durchgeführt wird.

2. Verfahren nach Anspruch 1, dadurch gekennzeichnet, daß das Substrat Glucose, Cholesterin, Glycerin, Glycerinphosphat, Cholin, Acyl CoA, Pyruvinsäure, Harsäurexanthin oder Milchsäure ist.

3. Verfahren nach Anspruch 1 oder 2, dadurch gekennzeichnet, daß die Probe mit einer Zusammensetzung behandelt wird, die (a) die genannte Oxidase, (b) die genannte Peroxidase oder die Eisenkomplexverbindung des Porphyrins oder das Eisenchelat des Komplexans, (c) die genannte Phenolverbindung und/oder das genannte Amin, (d) die genannte Thiolverbindung, (e) das genannte farberzeugende Reagens und (f) ein chelathildendes Mittel enthält.

4. Verfahren nach einem der vorhergehenden Ansprüche, dadurch gekennzeichnet, daß die genannte Probe mit dem genannten Amin behandelt wird, das Anilin, N-Ethylanilin, N,N-Dimethylanilin, N,N-Diethylanilin, N,N-Diethyl-m-toluidin, N-Ethyl-N-β-hydroxyethyl-m-toluidin, 1-N,N-Dimethyl-amino-4-naphthol oder 4-N,N-Diethylaminosalicylsäure ist.

5. Verfahren nach einem der vorhergehenden Ansprüche, dadurch gekennzeichnet, daß die genannte Probe mit der genannten Phenolverbindung behandelt wird, die Phenol, ein Chlorphenol, ein Dichlorphenol, ein Naphthol oder ein Sulfonsäurederivat ist.

6. Verfahren nach einem der vorhergehenden Ansprüche, dadurch gekennzeichnet, daß die Thiolverbindung reduziertes Glutathion, Thioglykolsäure, Mercaptoethanol, Thiosalicylsäure, Cysteamin, Cystein oder Dimercaptoberbsteinsäure ist.

7. Verfahren nach einem der vorhergehenden Ansprüche, dadurch gekennzeichnet, daß das farberzeugende Reagens ein Tetrazoliumsalz, Cytochrom C, Plastocyanin oder ein blaues Protein (Kupferprotein) ist.

8. Zusammensetzung für die quantitative Bestimmung eines Substrats mit (a) einer spezifischen

Oxidase, die einem zu bestimmenden Substrat entspricht, (b) einer Peroxidase, (c) einem Phenol und/oder einem Amin, (d) einer Thiolverbindung und (e) einem durch das Hyperoxidion reduzierbaren, farberzeugenden Reagens.

9. Zusammensetzung zur quantitativen Bestimmung eines Substrats mit (a) einer spezifischen Oxidase, die einem zu bestimmenden Substrat entspricht, (b') einer Eisenkomplexverbindung des Porphyrins oder einen Eisenchelat des Komplexans, (c) einem Amin und/der einem Phenol, (d) einer Thiolverbindung und (e) einem mit dem Hyperoxidion reduzierbaren, farberezugenden Reagens.

10. Zusammensetzung nach Anspruch 8 oder 9, die ferner (f) ein chelatbildendes Mittel enthält.

**Revendications**

1. Procédé pour déterminer un substrat, ce procédé consistant à traiter un échantillon contenant ce substrat avec une oxydase particulière correspondant à ce substrat pour donner naissance à un ion superoxyde, et à réduire un réactif produisant une couleur par cet ion superoxyde, caractérisé en ce que ce procédé est un procédé quantitatif qui met en jeu une mesure colorimétrique du réactif produisant de la couleur réduit et qui met en jeu en outre un traitement par une peroxydase ou un complexe de fer d'une porphyrine ou un chélate de fer de complexane, et un phénol et/ou une amine, et un thiol.

2. Procédé suivant la revendication 1, dans lequel le substrat est le glucose, le cholestérol, le glycérol, le phosphate de glycérol, la choline, l'acyl CoA, l'acide pyruvique, l'acide urique, la xanthine ou l'acide lactique.

3. Procédé suivant la revendication 1 ou 2, dans lequel cet échantillon est traité par une composition comprenant (a) cette oxydase, (b) cette peroxydase ou ce complexe de fer d'une porphyrine ou ce chélate de fer du complexane, (c) ce phénol et/ou cette amine, (d) ce thiol, (e) ce réactif produisant une couleur, et (f) un agent chélatant.

4. Procédé suivant l'une quelconque des revendications précédentes, dans lequel cet échantillon est traité par cette amine et cette amine est l'aniline, la N-éthylaniline, la N,N-diméthylaniline, la N,N-diéthylaniline, la N,N-diéthyl-m-toluidine, la N-éthyl-N-β-hydroxyéthyl-m-toluidine, la 1-N,N-diméthyl-amino-4-napthol, ou l'acide 4-N,N-diéthylaminosalicylique.

5. Procédé suivant l'une quelconque des revendications précédentes, dans lequel cet échantillon est traité par ce phénol et ce phénol est le phénol, un chlorophénol, un dichlorophénol, un napthol, ou un dérivé d'acide sulfonique.

6. Procédé suivant l'une quelconque des revendications précédentes, dans lequel ce thiol est du glutathion réduit, de l'acide thioglycolique, du mercaptoéthanol, de l'acide thiosalicylique, de la cystéamine, de la cystéine, ou de l'acide dimercaptosuccinique.

7. Procédé suivant l'une quelconque des revendications précédentes, dans lequel ce réactif produisant de la couleur est un sel de tétrazolium, le cytochrome C, la plastocyanine ou la protéine Bleue.

8. Composition pour la détermination quantitative d'un substrat comprenant (a) une oxydase particulière correspondant au substrat à déterminer, (b) une peroxydase, (c) un phénol et/ou une amine, (d) un thiol, et (e) un réactif produisant une couleur réductible par l'ion superoxyde.

9. Composition pour la détermination quantitative d'un substrat comprenant (a) une oxydase particulière correspondant au substrat à déterminer, (b') un complexe de fer d'une porphyrine ou un chélate de fer du complexane, (c) une amine et/ou un phénol, (d) un thiol et (e) un réactif produisant une couleur réductible par l'ion superoxyde.

10. Composition suivant la revendication 8 ou la revendication 9, qui comprend en outre (f) un agent chélatant.

FIG. 1

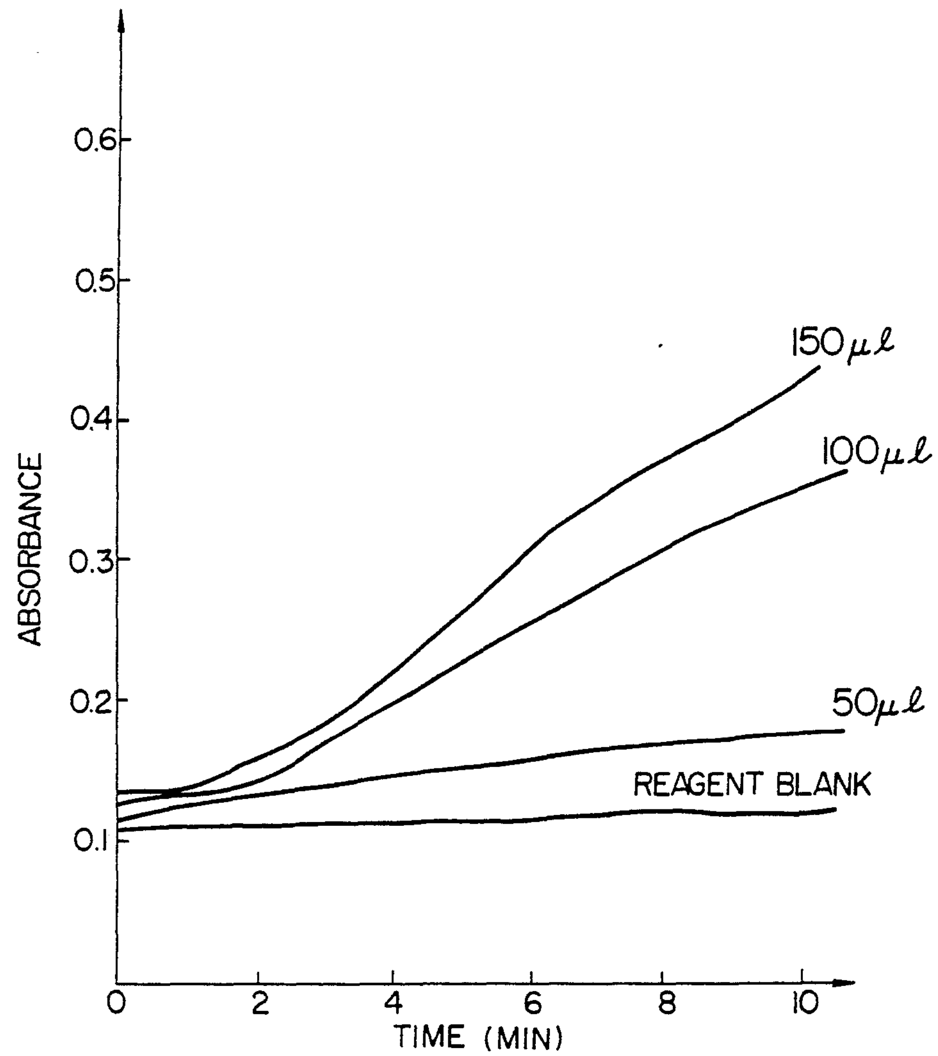
0.6
0.5
0.4
0.3
0.2
0.1
ABSORBANCE
150μl
100μl
50μl
REAGENT BLANK
0
2
4
6
8
10
TIME (MIN)

FIG. 2

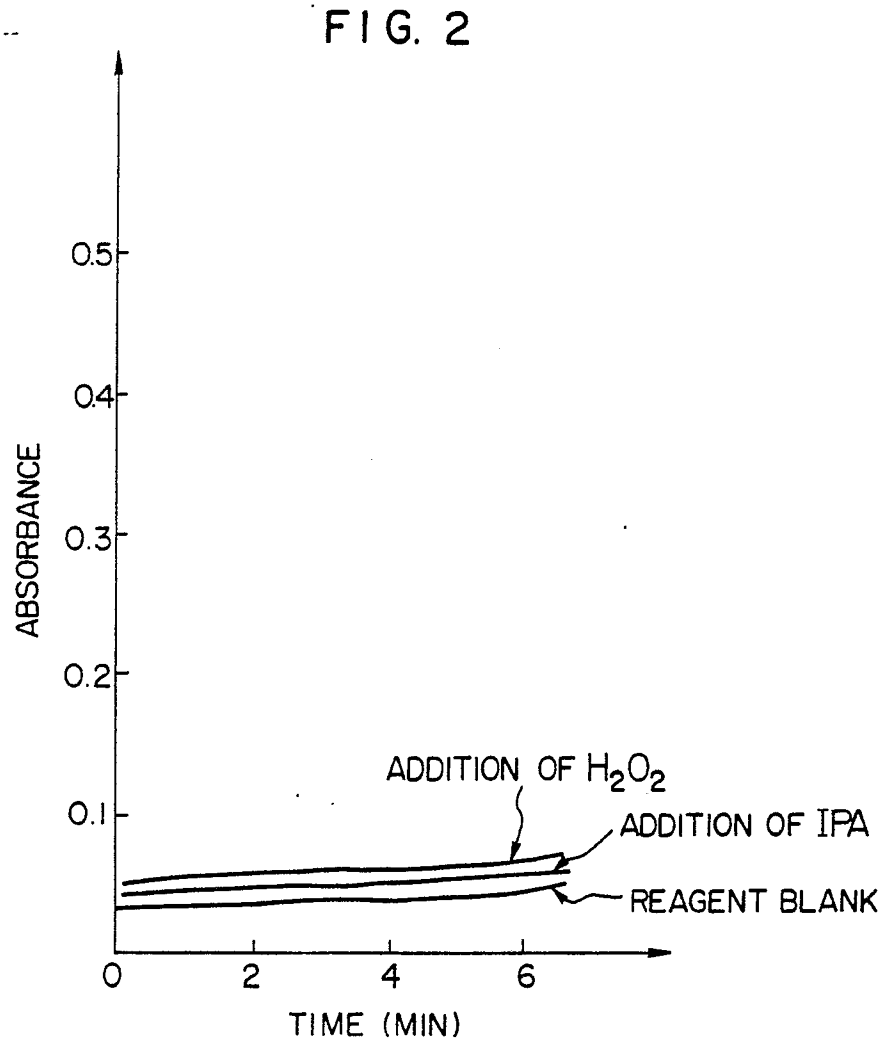
ABSORBANCE
0.5
0.4
0.3
0.2
0.1
ADDITION OF $H_2O_2$
ADDITION OF IPA
REAGENT BLANK
0
2
4
6
TIME (MIN)

FIG. 3

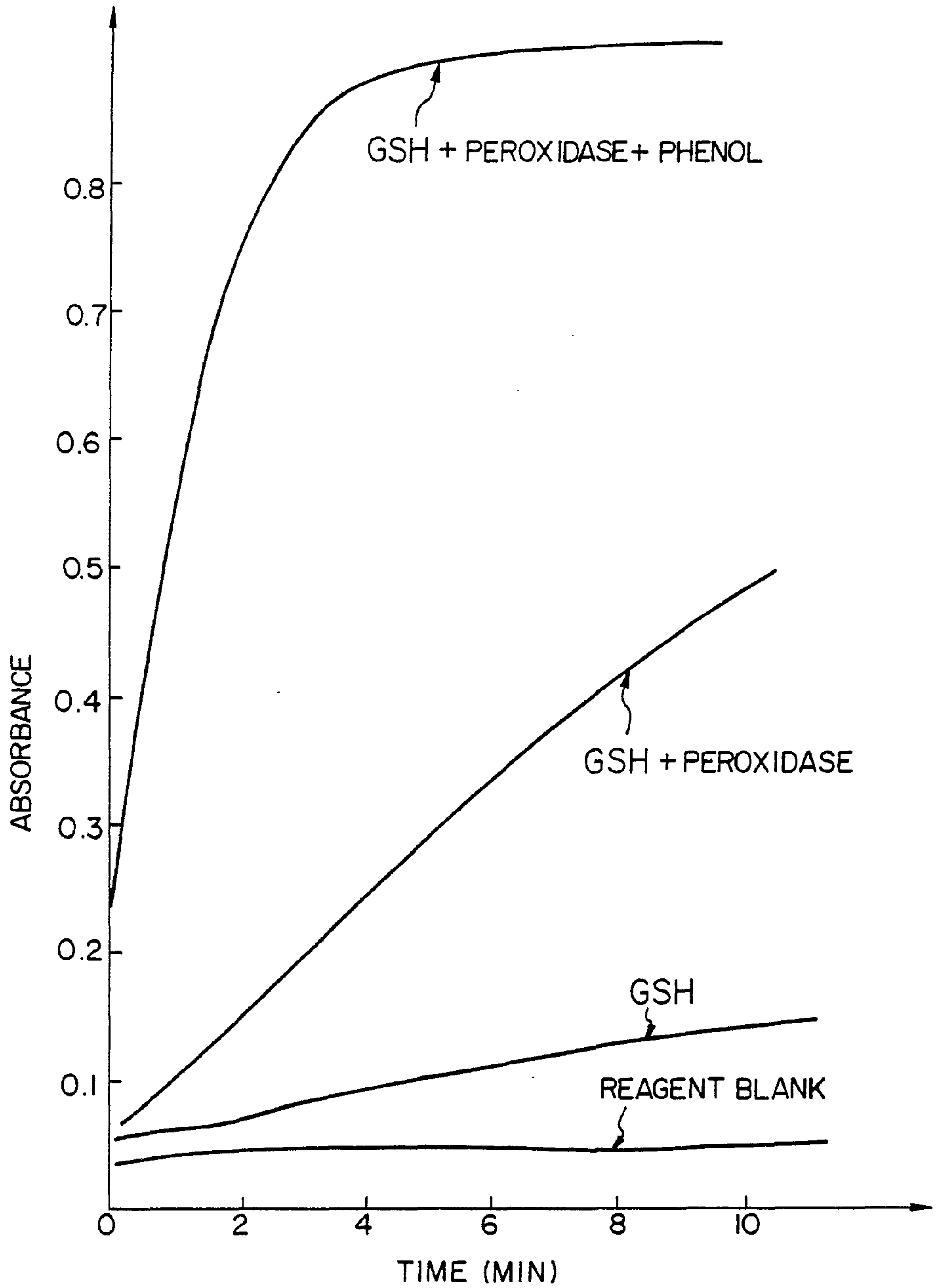
GSH + PEROXIDASE + PHENOL
GSH + PEROXIDASE
GSH
REAGENT BLANK
ABSORBANCE
0.8
0.7
0.6
0.5
0.4
0.3
0.2
0.1
0
2
4
6
8
10
TIME (MIN)

FIG. 4

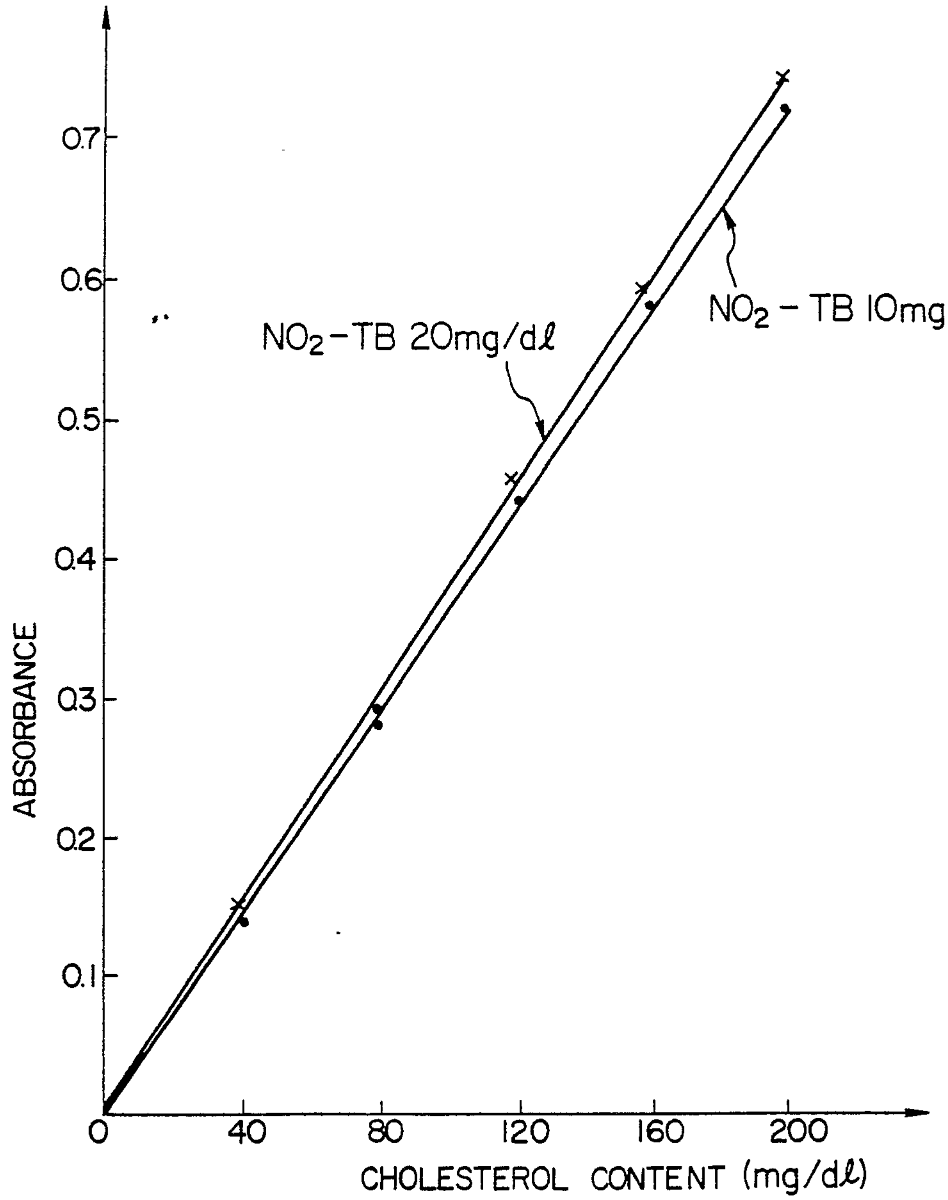

0.7
0.6
0.5
0.4
0.3
0.2
0.1
ABSORBANCE
0
40
80
120
160
200
CHOLESTEROL CONTENT (mg/dℓ)
NO2-TB 20mg/dℓ
NO2 - TB 10mg/dℓ

FIG. 5